# EUROPEAN PATENT APPLICATION

(11) **EP 2 606 923 A1**
(43) Date of publication of application: **26.06.2013**
(21) Application number: 12198447.0
(22) Date of filing: 20.12.2012
(51) Int. Cl.: A61M 5/158, A61M 5/32

(54) **Safety intravenous needle set**

(30) Priority: 20.12.2011 TW 100224058
(71) Applicant: Sunwell Global Limited, Tortola (VG)
(72) Inventor: Li, Zhi-Yun, Nantong City (CN)
(74) Representative: Baldwin, Mark

(57) **Abstract**

A safety intravenous needle set includes needle hub (23) holding a needle (11) and an infusion tube (25) at two opposite ends thereof, and a safety device (27), which includes a base block (270) affixed to the needle hub, a recessed guard (271) pivoted to the base block and having a retaining lug (273) located on one of two opposing inner wall thereof. The recessed guard is biasable relative to the needle hub toward the medical needle to let the medical needle be forced over the retaining lug into the inside of the recessed guard and then retaining inside the recessed guard by the retaining lug, avoiding accidental needle stick injury after the service of the safety intravenous needle set.

## Description

### BACKGROUND OF THE INVENTION

The present invention is related to a safety intravenous needle set, which is equipped with an anti-piercing safety device to prevent the needle from piercing the healthcare worker accidentally.

A healthcare worker many need to inject a medicine into the body of a patient or to draw blood from a blood vessel of a patient by means of an intravenous needle. However, when handling an intravenous needle during a medical procedure, the healthcare worker may be injured by the needle accidentally.

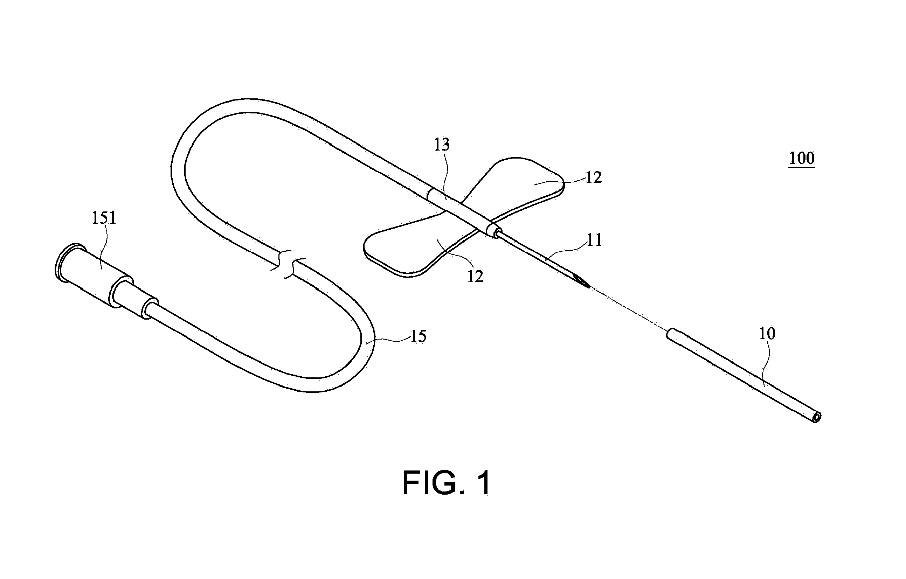

Fig. 1 illustrates a conventional intravenous needle set. As illustrated, the intravenous needle set **100** comprises a needle cap **10,** a medical needle **11,** a needle hub **13,** and an infusion tube **15.**

The needle cap **10** is detachably capped on the medical needle **11** for protection before the use of the intravenous needle set **100.** The infusion tube **15** is a fluid passage for intravenous infusion or drawing blood, having its one end connected to the needle hub **13** and its other end provided with a connector **151** for the connection of a feeding tube of an infusion bag, saline bag or blood bag, or a medical device. Further, the needle hub **13** has two wings **12** arranged at two opposite lateral sides thereof.

When using the intravenous needle set **100,** the healthcare worker can remove the needle cap **10** from the medical needle **11** to expose the medical needle **11.** The healthcare worker can conveniently insert the medical needle **11** into the patient's vein, blood vessel or body and then secure the wings **12** to the patient's skin by a medical adhesive tape. At this time, the healthcare worker can start an intravenous infusion or drawing blood.

After use, the healthcare worker will remove the medical needle **11** from the body of the patient and then cap the needle cap **10** on the medical needle **11** again. However, as the needle cap **10** defines therein a small cap hole, it is difficult to accurately cap the needle cap **10** onto the medical needle **11.** This may result in accidental injury to the healthcare worker by the medical needle **11,** leading to a higher risk of virus infection (AIDS, HIV SARS, etc.). Handling an intravenous needle set in this manner is dangerous to healthcare workers.

Therefore, there is a strong demand for a safety intravenous needle set, which enhances safe needle disposal, preventing infections in healthcare workers due to accidental needle injury.

### SUMMARY OF THE PRESENT INVENTION

It is an object of the present invention to provide a safety intravenous needle set, which is equipped with a safety device that can receive the medical needle after use of the safety intravenous needle set so as to reduce the likelihood of accidental needle stick injury or to at least provide an alternative to known intravenous needle sets.

The present invention may provide a safety intravenous needle set, which enables the medical needle to be accommodated inside a safety device after its service, thereby protecting waste needle handling persons against accidental needle stick injury and avoiding a repeat use of the medical needle.

The present invention may provide a safety intravenous needle set, comprising: a needle hub holding a medical needle at one end thereof and an infusion tube at an opposite end thereof; and a safety device, said safety device comprising a base block, a recessed guard and a pivot, said recessed guard comprises two opposing inner walls, an outer wall and at least one pair of retaining lugs located on said two opposing inner walls, each said pair of retaining lugs defining therebetween a constraint gap, said base block being fixedly mounted at said needle hub, said recessed guard being pivotally connected to said base block by said pivot for allowing said recessed guard to be biased relative to said base block and said needle hub toward said medical needle to let said medical needle be forced through said constraint gap into the inside of said recessed guard.

In one embodiment of the present invention, said recessed guard has a relatively narrower top side and a relatively wider bottom side so that said outer wall defines a bevel face at each of two opposite lateral sides thereof.

In one embodiment of the present invention, said recessed guard comprises an anti-slip structure located on said outer wall.

In one embodiment of the present invention, said anti-slip structure comprises at least one longitudinal rib, at least one transverse rib, at least one raised portion, at least one recessed portion, at least one finger notch and/or at least one finger retaining means.

In one embodiment of the present invention, said needle hub comprises a wing located on at least one of two opposite lateral sides thereof.

In one embodiment of the present invention, said retaining lugs of said safety device are arranged in a staggered manner, defining a longitudinal pitch between each two adjacent said retaining lugs.

In one embodiment of the present invention, said constraint gap has a width equal to or smaller than the diameter of said medical needle.

In one embodiment of the present invention, the other end of said infusion tube is connected to a connector.

In one embodiment of the present invention, the connector is provided with a transfixion needle connected to the infusion tube and the length of the connector is longer than the length of the transfixion needle.

The present invention may provide a safety intravenous needle set, comprising: a needle hub holding a medical needle at one end thereof and an infusion tube at an opposite end thereof; and a safety device, said safety device comprising a base block, a recessed guard and a pivot, said recessed guard comprises two opposing inner walls, an outer wall and at least one retaining lug located on one said inner wall, said base block being fixedly mounted at said needle hub, said recessed guard being pivotally connected to said base block by said pivot for allowing said recessed guard to be biased relative to said base block and said needle hub toward said medical needle to let said medical needle be forced over said at least one retaining lug into the inside of said recessed guard and then retaining inside said recessed guard by said at least one retaining lug.

In one embodiment of the present invention, each said retaining lug has a length greater than one half of the internal width of said recessed guard.

In one embodiment of the present invention, the other end of said infusion tube is connected to a connector, said connector is provided with a transfixion needle connected to the infusion tube and the length of the connector is longer than the length of the transfixion needle.

### BRIEF DESCRIPTION OF THE DRAWINGS

Fig. 1 is a schematic perspective view of an intravenous needle set according to the prior art.
Fig. 2 is a schematic perspective view of a safety intravenous needle set in an opened status in accordance with a first embodiment of the present invention.
Fig. 3 corresponds to Fig. 2, illustrating the safety intravenous needle set in a closed status.
Fig. 4 is a bottom view of a part of the safety intravenous needle set shown in Fig. 3.
Fig. 4A is an enlarged view of a part of Fig. 4.
Fig. 5 is a cross sectional view, in an enlarged scale, of a part of the safety intravenous needle set shown in Fig. 3.
Fig. 6 is a schematic cross sectional view of a safety intravenous needle set in a closed status in accordance with a second embodiment of the present invention.
Fig. 7 is a schematic perspective view of a safety intravenous needle set in accordance with a third embodiment of the present invention.
Fig. 8 is a bottom view of a part of a safety intravenous needle set in accordance with a fourth embodiment of the present invention.
Fig. 8A is an enlarged view of a part of Fig. 8.
Fig. 9 is a schematic cross sectional view, in an enlarged scale, of the safety device of the safety intravenous needle set in accordance with the fourth embodiment of the present invention.
Fig. 10 corresponds to Fig. 8, illustrating an alternate form of the arrangement of the retaining lugs in the recessed guard of the safety device.
Fig. 10A is an enlarged view of a part of Fig. 10.
Fig. 11 illustrates a safety intravenous needle set in accordance with a fifth embodiment of the present invention.
Fig. 12 illustrates an alternate form of the anti-slip structure of the safety device of the safety intravenous needle set in accordance with the fifth embodiment of the present invention.
Fig. 13 illustrates another alternate form of the anti-slip structure of the safety device of the safety intravenous needle set in accordance with the present invention.
Fig. 14 illustrates another alternate form of the anti-slip structure of the safety device of the safety intravenous needle set in accordance with the present invention.

### DETAILED DESCRIPTION OF THE ILLUSTRATED EMBODIMENTS

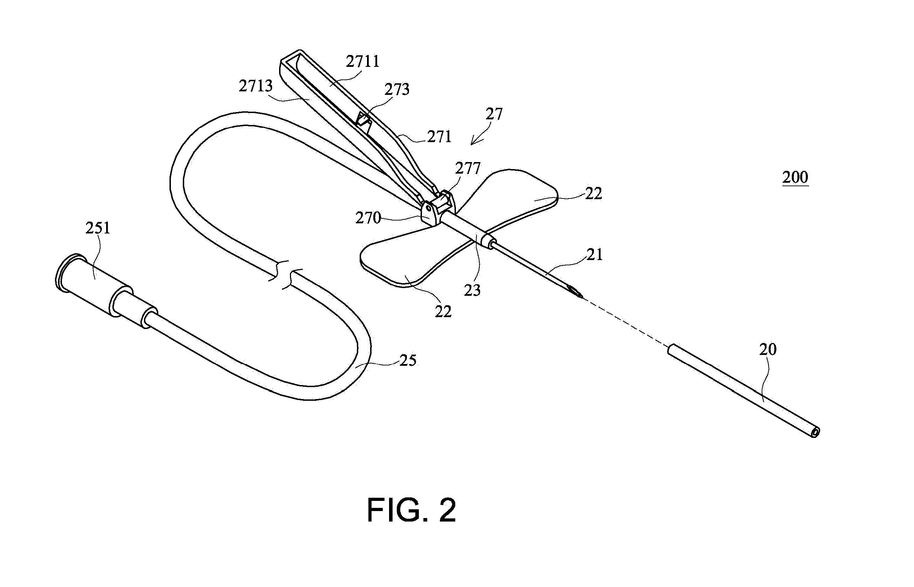

Referring to Figs. 2 through 5, a safety intravenous needle set **200** comprises a medical needle **21,** a needle hub **23,** an infusion tube **25** and a safety device **27.**

The needle hub **23** has one end connected with the medical needle **21** and its other end connected with the infusion tube **25.** Further, the needle hub **23** has two wings **22** arranged at two opposite lateral sides thereof. A needle cap **20** is detachably capped, or fitted, on the medical needle **21** for protection.

The infusion tube **25** is a fluid passage for intravenous infusion or drawing blood, having its one end connected to the needle hub **23** and its other end provided with a connector **251** for the connection of a feeding tube of an infusion bag, saline bag or blood bag, or a medical device.

The safety device **27** is an anti-piercing device, comprising a base block **270,** a recessed guard **271,** and a pivot **277.** The recessed guard **271** comprises two inner walls **2711,** an outer wall **2713,** and at least one retaining lug **273** located on one inner wall **2711.** The base block **270** is mounted at the needle hub **23.** The recessed guard **271** is pivotally connected to the base block **270** by the pivot **277.** By means of the pivot **277,** the recessed guard **271** is movable relative to the base block **270.**

Before the use of the safety intravenous needle set **200** shown in Fig. 2, the safety device **27** is opened from the medical needle **21.** In the illustrated example, this involves moving the recessed guard **271** from a position in which the medical needle **21** is received within the recessed guard to a position, such as that shown in Fig. 2, in which the medical needle is exposed for use. When using the safety intravenous needle set **200,** the healthcare worker can remove the needle cap **20** from the medical needle **21** to expose the medical needle **21.** The healthcare worker can insert the medical needle **21** into the patient's vein, blood vessel or body and then conveniently secure the wings **22** to the patient's skin by a medical adhesive tape. At this time, the healthcare worker can start an intravenous infusion or drawing blood from the patient.

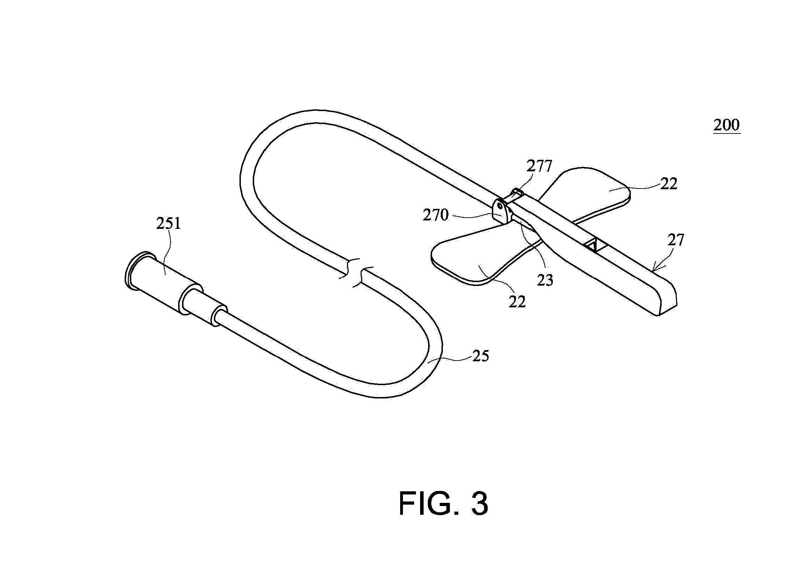

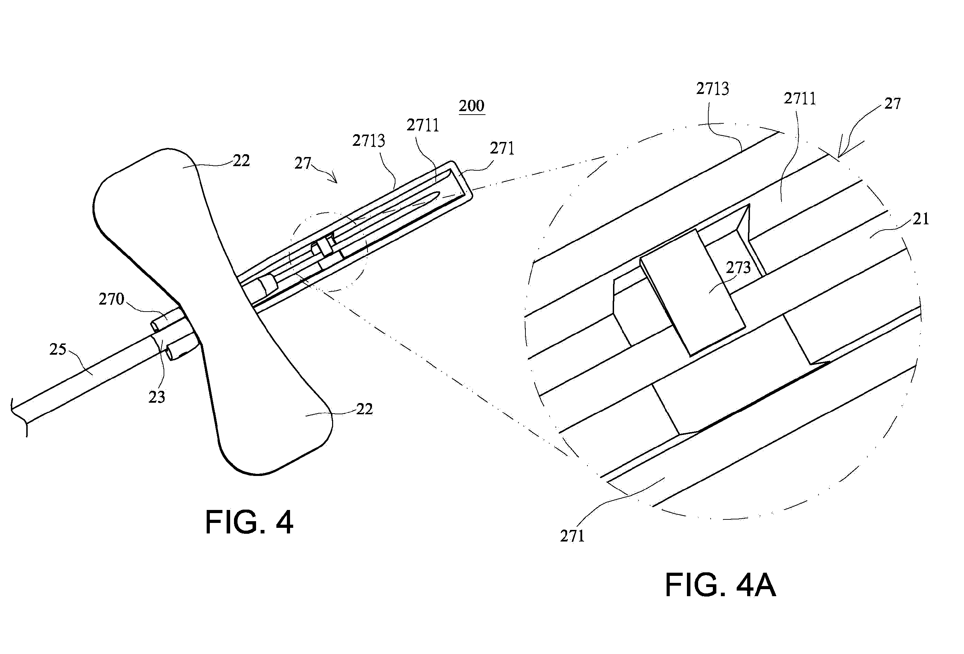

Once a procedure is complete, the healthcare worker will remove the medical needle **21** from the body of the patient. As illustrated in Fig. 3, Fig. 4 and Fig. 4A, when going to dispose of the used medical needle **21,** the healthcare worker can turn the recessed guard **271** about the pivot **277** towards the top side of the medical needle **21** and then press the recessed guard **271** down or push the medical needle **21** upwardly, to force the medical needle **21** into the inside of the recessed guard **271.** At this time, the medical needle **21** is secured inside the recessed guard **271** by the retaining lug **273.** Thus, the safety device **27** and the medical needle **21** are secured together in the closed status, and the used medical needle **21** is well protected inside the safety device **27** for further medical waste treatment.

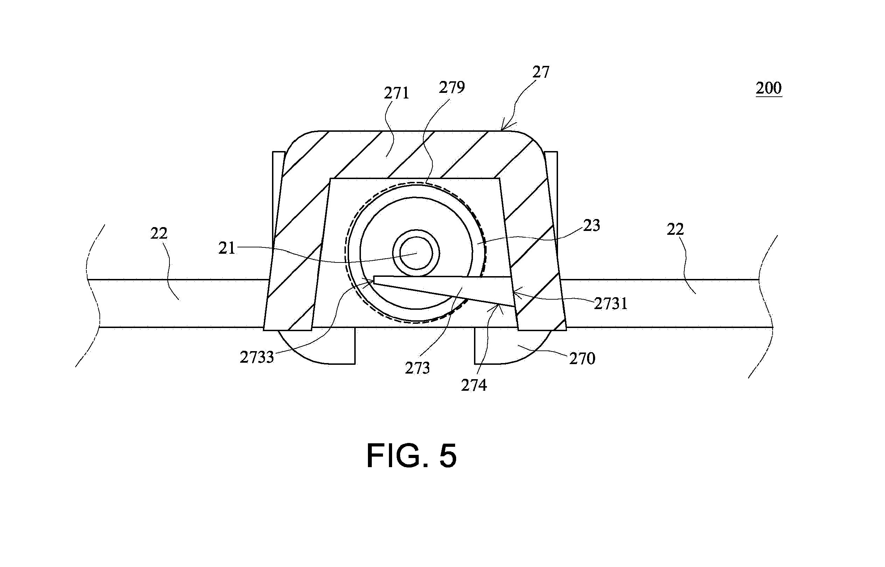

Referring to Fig. 5, the retaining lug **273** has a connection end **2731** and an oppositely disposed retaining end **2733.** By means of the connection end **2731,** the retaining lug **273** is affixed to one inner wall **2711** of the recessed lug **271.** The connection end **2731** has a relatively larger thickness than the retaining end **2733.** Further, the retaining lug **273** defines a bevel, or inclined, face **274** on one side thereof (the outer side) between the connection end **2731** and the retaining end **2733.** Subject to the arrangement of the bevel face **274,** the medical needle **21** can be moved along the bevel face **274** into the inside of the recessed guard **271** easily. When pushed by an external pressure, the medical needle **21** can be easily moved into the gap between the retaining lug **273** and the recessed guard **271** with less effort.

In one embodiment of the present invention, the base block **270** of the safety device **27** comprises a hollow passage **279** (see the imaginary line in Fig. 5), or a C-shaped ring, for the insertion and affixation of the needle hub **23** of the safety intravenous needle set **200** so that the base block **270** can be mounted at the needle hub **23.**

Further, if only one single retaining lug **273** is provided, the length of the retaining lug **273** should be greater than one half of the internal width of the recessed guard **271,** i.e., the retaining end **2733** will extend across the centre line of the recessed guard **271** where the medical needle **21** is to be accommodated. When forcing the medical needle **21** toward the inside of the recessed guard **271,** the retaining lug **273** will be forced to curve inwardly, or deflect, by the medical needle **21,** and the medical needle **21** will be forced to displace sideways and then to enter the gap between the retaining lug **273** and the recessed guard **271.**

After the medical needle **21** has entered the recessed guard **271,** the medical needle **21** and the retaining lug **273** immediately return to their former position or shape so that the medical needle **21** can be positively positioned in the middle position inside the recessed guard **271** and the retaining end **2733** of the retaining lug **273** can transversely extend over the medical needle **21.** Under normal impact or external pressure, the medical needle **21** will not be forced to displace sideways or to move out of the recessed guard **271.** Thus, the medical needle **21** can be securely accommodated in the recessed guard after use, thereby avoiding a repeat use.

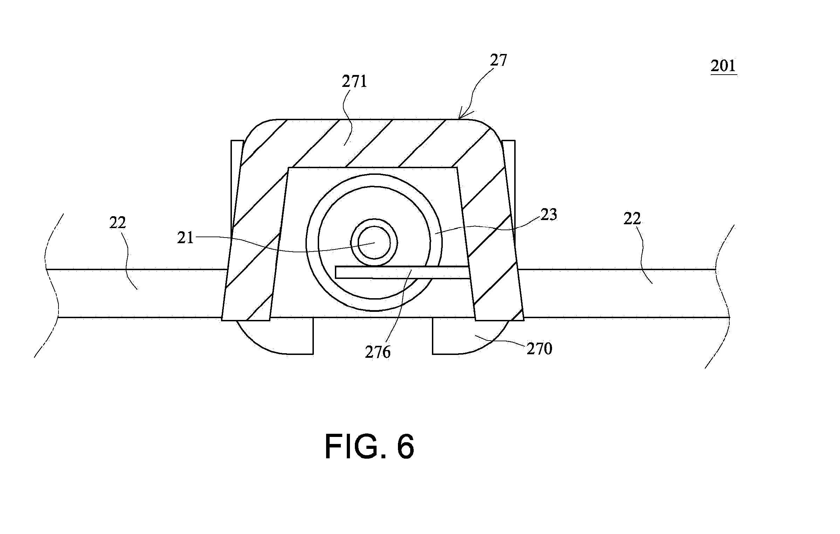

Fig. 6 illustrates a safety intravenous needle set **201** in accordance with a second embodiment of the present invention. According to this second embodiment, the retaining lug **276** is a planar thin sheet member without the aforesaid bevel face **(274).** This thin planar sheet member retaining lug **276** is elastically and inwardly bendable so that the medial needle **21** can be moved along the elastically and inwardly deformed curvature of the retaining lug **276** into the inside of the recessed guard **271.**

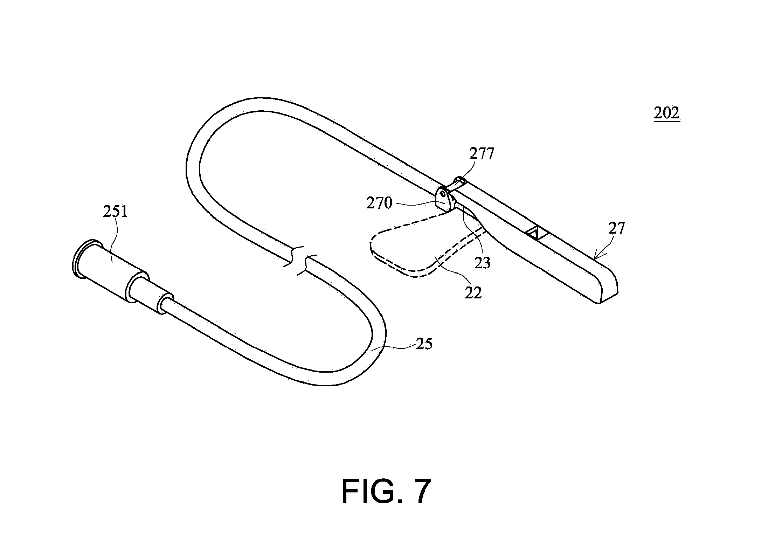

Further, as shown in Fig. 7, illustrates a safety intravenous needle set **202** in accordance with a third embodiment of the present invention. According to this third embodiment, the needle hub **23** has only one wing **22** located on one lateral side thereof. The other lateral side of the needle hub **23** is kept clean. In order to facilitate manipulation of the safety intravenous needle set **202** by the healthcare worker, the thickness of the single wing **22** may be made relatively thicker that the double wing design in the aforesaid first and second embodiments.

It is to be understood that the needle hub **23** can be made without any wings **22.** A safety intravenous needle set **202** with no wings **22** considerably reduces the manufacturing cost and is applicable for use as a regular injection syringe needle, for example, an intramuscular injection syringe needle.

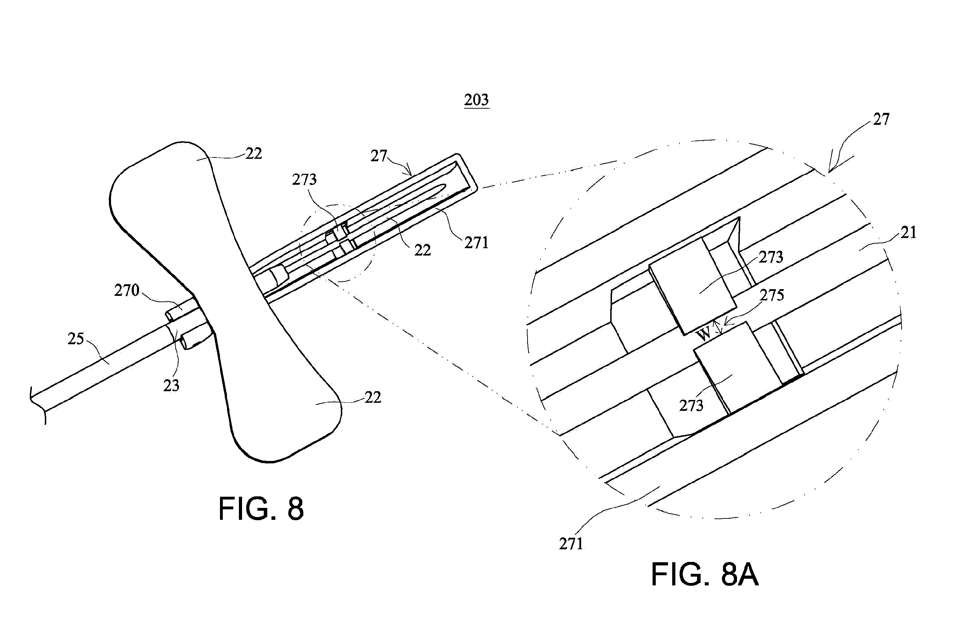

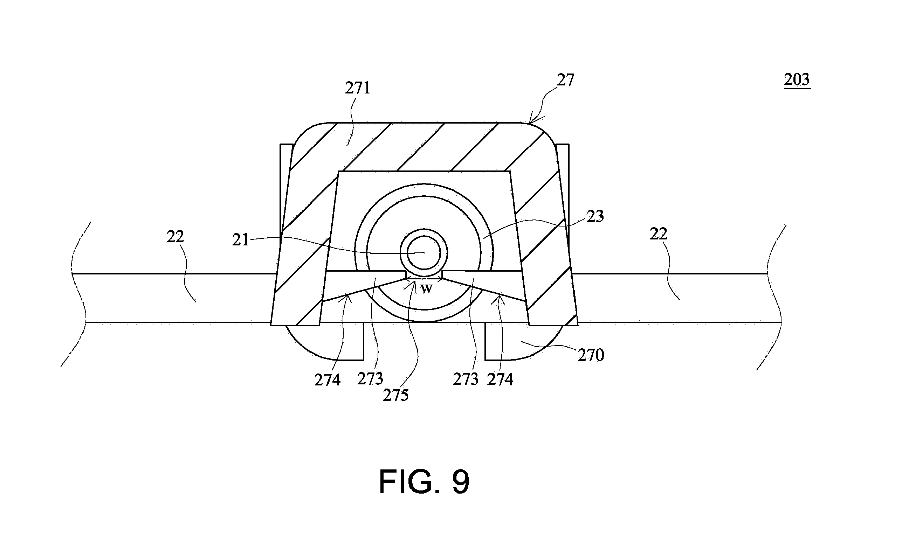

Fig. 8, Fig. 8A and Fig. 9 illustrate a safety intravenous needle set **203** in accordance with a fourth embodiment of the present invention. In the first, second or third embodiments, the recessed guard has one retaining lug **273** or **276** located on only one inner wall thereof. According to this fourth embodiment, the recessed guard **271** has at least one retaining lug **273** or **276** located on each of the two inner walls thereof. Each retaining lug **273** or **276** can be configured to provide a bevel face **274,** or be in the shape of a thin planar sheet member.

Further, a constraint gap **275** is defined between the two retaining lugs **273** or **276** (or each pair of retaining lugs) that are symmetrically arranged at the two inner walls of the recessed guard **27.** The constraint gap **275** has a width **W** approximately equal to the diameter of the medical needle **21** so that the medical needle **21** cannot be easily forced out of the recessed guard **27** through the constraint gap **275.** Preferably, the width **W** of the constraint gap **275** is smaller than the diameter of the medical needle **21.**

When the healthcare worker goes to cover the used medical needle **21,** the recessed guard **271** of the safety device **27** is moved towards the medical needle **21,** which is then forced through the constraint gap **275** into the inside of the recessed guard **271.** This enables the used medical needle **21** to be held inside the recessed guard **271** by the retaining lugs **273.** Thus, the retaining lugs **273** hold the used medical needle **21** inside the recessed guard **271,** avoiding both exposure of the used medical needle **21** to the outside of the recessed guard **271** and repeated use of the safety intravenous needle set **203.**

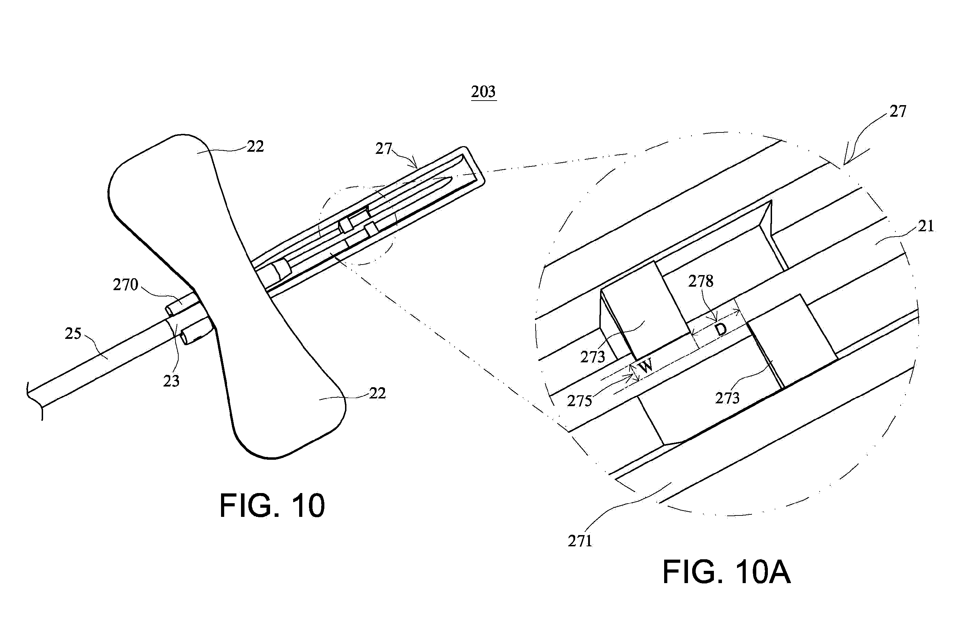

Further, as shown in Fig. 10 and Fig. 10A, the retaining lugs **273** of the safety intravenous needle set **203,** unlike the symmetrical arrangement shown in Fig. 8, can be arranged in a staggered manner with a longitudinal pitch **(D) 278** defined therebetween. In this case, one retaining lug **273** can retain a front part of the medical needle **21** and the other retaining lug **273** can retain a rear part of the medical needle **21,** enhancing the positioning stability of medical needle **21** in the recessed guard **27.**

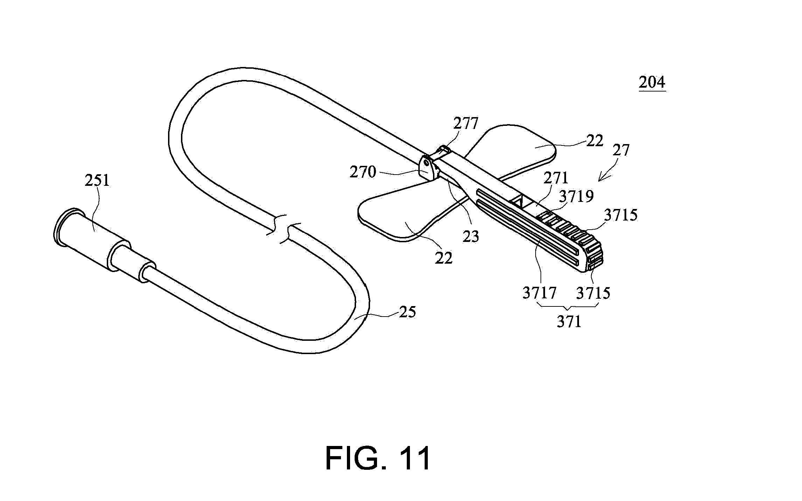

Fig. 11 illustrates a safety intravenous needle set **204** in accordance with a fifth embodiment of the present invention. After using the safety intravenous needle set **204,** the healthcare worker normally will move the recessed guard **271** of the safety device **27** towards the medical needle **21.** At this time, improper posture or improper finger positioning on the recessed guard **271** may cause the user's fingers to slip over the recessed guard **271** and to be injured by the medical needle **21.** To avoid this problem, an anti-slip structure **371** is located on the outer wall **2713** of the recessed guard **271.** According to this embodiment, the anti-slip structure **371** comprises a plurality of transverse ribs **3715** located on the top side of the outer wall **2713** of the recessed guard **271,** and a plurality of longitudinal ribs **3717** located on two opposite lateral sides of the outer wall **2713.**

When the healthcare worker or any other user is going to dispose of the used medical needle **21,** hold the outer wall **2713** is held with the fingers and then the recessed guard **271** is moved towards the medical needle **21.** Due to the anti-slip effect of the anti-slip structure **371,** the healthcare worker or user can hold the outer wall **2713** of the recessed guard **271** with the fingers positively, avoiding finger slipping or accidental needle stick injury.

It is to be understood that the anti-slip structure **371** is not limited to the transverse ribs **3715** and/or longitudinal ribs **3717.** Alternatively, the anti-slip structure **371** can be formed of raised portions, or longitudinally and/or transversely extending grooves **3719,** achieving the safe anti-slip effect.

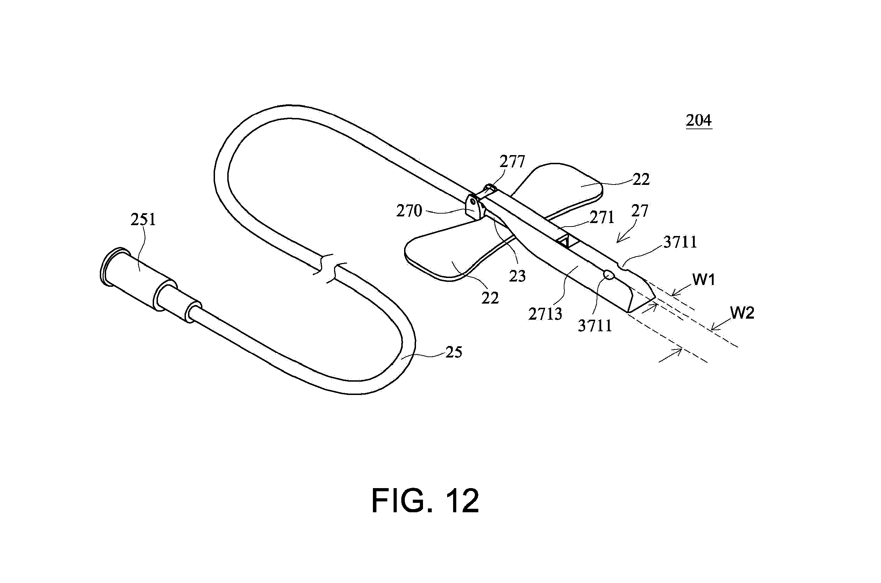

Further, as shown in Fig. 12, the anti-slip structure of the safety device **27** of the safety intravenous needle set **204** can comprise at least one finger notch **3711** on the outer wall **2713.** When moving the safety device **27,** the healthcare worker or user can rest the thumb and/or forefinger in the at least one finger notch **3711** of the recessed guard **271** and then move the recessed guard **271.**

As the user's thumb and/or forefinger is rests in the at least one finger notch **3711** of the recessed guard **271** when holding the recessed guard **271,** the user's thumb and/or forefinger is (are) obstructed from displacement relative to the recessed guard **271,** avoiding accidental needle stick injury.

Further, the width **W1** of the top side and the width **W2** of the bottom side of the recessed guard **271** are unequal. The width **W2** of the bottom side of the recessed guard **271** is greater than the width **W1** of the top side of the recessed guard **271.** Thus, the two opposite lateral sides of the outer wall **2713** of the recessed guard **271** of the safety device **27** provide respective bevel, or inclined, faces. If the user's fingers slip away when moving the recessed guard **271** of the safety device **27,** the bevel faces of the outer wall **2713** of the recessed guard **271** guide the user's fingers away from the effective range of the medical needle **21,** assuring a high level of safety of the use of the safety intravenous needle set **204.**

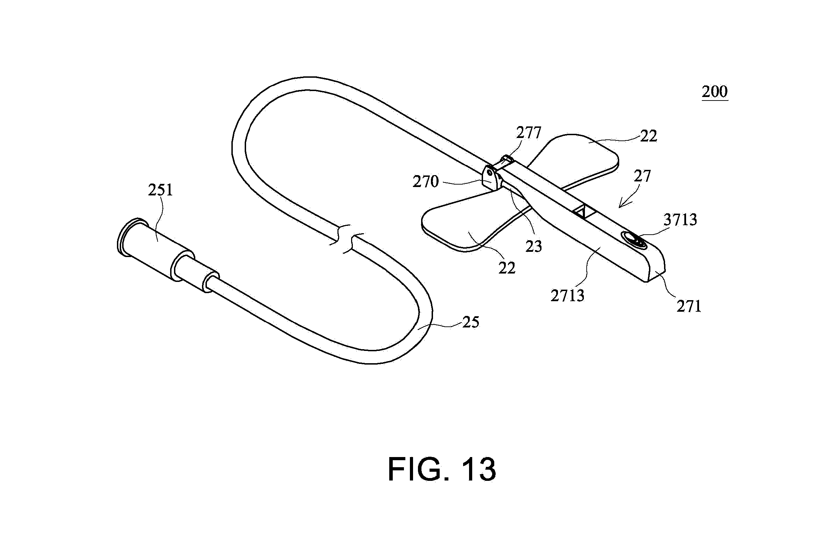

Further, Fig. 13 illustrates another alternate form of the anti-slip structure. According to this embodiment, the anti-slip structure comprises a finger retaining means **3713** located on the outer wall **2713** of the recessed guard **271** of the safety device **27** of the safety intravenous needle set **200.** The finger retaining means **3713** can be a recessed portion or raised portion with ribs or grooves therein. The anti-slip structure **3713** can obstruct displacement of the user's fingers relative to the recessed guard **271,** avoiding accidental needle stick injury.

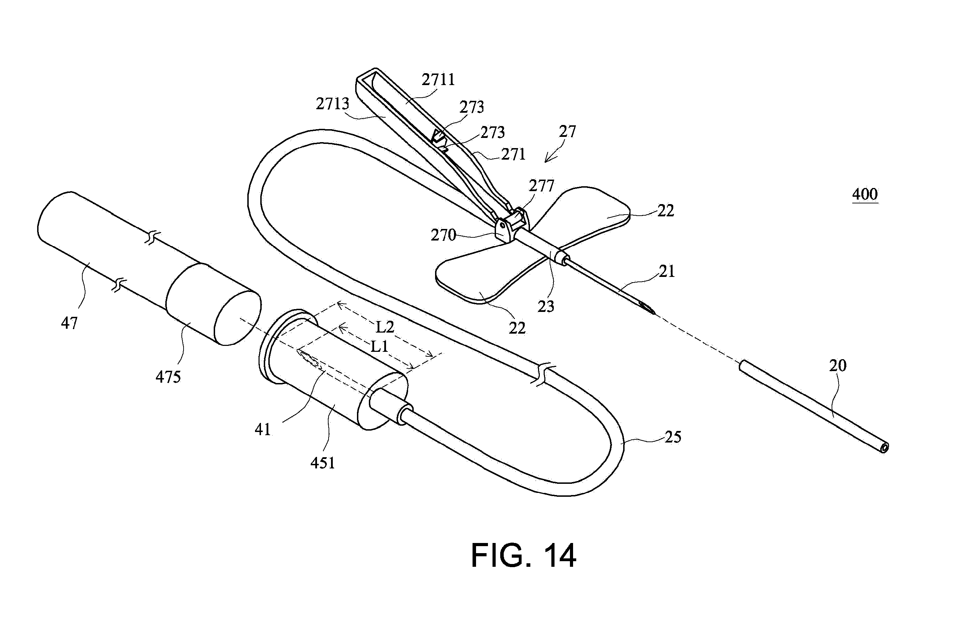

Fig. 14 illustrates a safety intravenous needle set **400** in accordance with an embodiment of the present invention. The safety intravenous needle set 400 can be used for blood lancing device or a safety infusion needle set. In the present embodiment, one end of the infusion tube 25 is connected to the needle hub 23, the other end of the infusion tube 25 is connected to a connector 451. The connector 451 is provided with a transfixion needle 41 for piercing a stopper 475 of a blood collection tube 47. Blood can flow into the blood collection tube 47 via the medical needle 21, the infusion tube 25, and the transfixion needle 41.

When the blood collection tube 47 is filled with enough blood or body fluid, the blood collection tube 47 is pulled out of the connector 451. Since the length L2 of the connector 451 is longer than the length L1 of the transfixion needle 41, the transfixion needle 41 with blood or body fluid is retained in the connector 451 for avoiding accidental needle stick injury. The medical needle 21 at the front of the safety intravenous needle set 400 is also secured by the safety device 27.

In conclusion, the embodiments provide safety intravenous needle sets **200/201/202/203/204** equipped with a safety device **27.** After the use of the medical needle **21,** the recessed guard **271** of the safety device **27** can be moved towards to the medical needle **21** and the medical needle **21** forced into the recessed guard **271** and secured therein, thereby avoiding accidental needle stick injury or a repeat use of the safety intravenous needle set and assuring a high level of medical waste disposal safety.

Embodiments of the invention provide an intravenous needle set comprising a needle holder, a needle held by said needle holder and a safety guard connected with said needle holder, said safety guard being movable between a position in which said needle is exposed for use and a position in which said needle is received in said guard.

Embodiments of the invention provide a method of disposing of a used intravenous needle set that comprises a needle holder, a needle held by said needle holder and safety guard connected with the needle holder, said method comprising moving the safety guard from a position in which the needle is exposed for use and a position in which the needle is received in said guard.

The foregoing description is merely of example embodiments of the present invention and not to be considered as restrictive. All equivalent variations and modifications in shape, structure and feature may be made without departing from the scope of the claims.

## Claims

1. A safety intravenous needle set comprising:
a needle hub (23) holding a medical needle and connected with an infusion tube (25); and
a safety device (27), said safety device comprising a base block (270), a recessed guard (271) and a pivot (277), said recessed guard comprising two opposing inner walls (2711), an outer wall (2173) and at least one pair of retaining lugs (273; 276) located on said two opposing inner walls, each said pair of retaining lugs defining therebetween a constraint gap (275), said base block being fixedly mounted at said needle hub, said recessed guard being pivotally connected to said base block by said pivot for allowing said recessed guard to be moved relative to said base block and said needle hub toward said medical needle to let said medical needle be forced through said constraint gap into said recessed guard.

2. A safety intravenous needle set as claimed in Claim 1, wherein said recessed guard has a relatively narrower (W1) top side and a relatively wider (W2) bottom side so that said outer wall defines respective inclined side faces extending between said top and bottom sides.

3. A safety intravenous needle set as claimed in Claim 1 or 2, wherein said recessed guard (271) comprises an anti-slip structure (371; 3713) located on said outer wall (2713).

4. A safety intravenous needle set as claimed in Claim 3, wherein said anti-slip structure comprises at least one longitudinal rib (3717), at least one transverse rib (3715), at least one raised portion, at least one recessed portion (3719), at least one finger notch (3711) and/or at least one finger retaining means.

5. A safety intravenous needle set as claimed in any one of the preceding Claims, wherein at least one of two sides of said needle hub (23) has a wing (22) extending therefrom.

6. A safety intravenous needle set as claimed in any one of the preceding Claims, wherein said constraint gap (275) has a width equal to or smaller than the diameter of said medical needle (21).

7. A safety intravenous needle set as claimed in any one of the preceding Claims 1, wherein said infusion tube has an end connected with a connector (251; 451).

8. A safety intravenous needle set as claimed in Claim 7, wherein the connector (451) is provided with a transfixion needle (41) connected to the infusion tube, the length of the connector being longer than the length of the transfixion needle.

9. A safety intravenous needle set, comprising:
a needle hub (23) holding a medical needle and connected with an infusion tube (25); and
a safety device (27), said safety device comprising a base block (270), a recessed guard (271) and a pivot (277), said recessed guard comprising two opposing inner walls (2711), an outer wall (2173) and at least one retaining lug (273; 276) located on one said inner wall, said base block being fixedly mounted at said needle hub, said recessed guard being pivotally connected to said base block by said pivot for allowing said recessed guard to be move relative to said base block and said needle hub toward said medical needle to let said medical needle be forced past said at least one retaining lug into said recessed guard to be retained in said recessed guard by said at least one retaining lug.

10. A safety intravenous needle set as claimed in Claim 9, wherein said recessed guard has a relatively narrower (W1) top side and a relatively wider (W2) bottom side so that said outer wall defines respective inclined faces extending between said top and bottom sides.

11. A safety intravenous needle set as claimed in Claim 9 or 10, wherein said recessed guard (27) comprises an anti-slip structure (371; 3713) located on said outer wall (2713).

12. A safety intravenous needle set as claimed in Claim 11, wherein said anti-slip structure comprises at least one longitudinal rib (3717), at least one transverse rib (3715), at least one raised portion, at least one recessed portion (3719), at least one finger notch (3711) and/or at least one finger retaining means.

13. A safety intravenous needle set as claimed in any one of Claims 9 to 12, wherein at least one of two opposite sides of said needle hub (23) has a wing (22) extending therefrom.

14. A safety intravenous needle set as claimed in any one of Claims 9 to 13, wherein the or each said retaining lug (273; 276) has a length greater than one half of the internal width of said recessed guard.

15. A safety intravenous needle set as claimed in any one of Claims 9 to 14, wherein an end of said infusion tube (25) is connected to a connector (451), said connector is provided with a transfixion needle (41) connected to the infusion tube, and the length of the connector is longer than the length of the transfixion needle.
